(19) Europäisches Patentamt
European Patent Office
Office européen des brevets

(11) Publication number: **0 382 368 A1**

(12) **EUROPEAN PATENT APPLICATION**

(21) Application number: **90300650.0**

(51) Int. Cl.5: **C07D 239/26, //A01N43/54**

(22) Date of filing: **22.01.90**

(30) Priority: **07.02.89 GB 8902688**

(43) Date of publication of application:
**16.08.90 Bulletin 90/33**

(84) Designated Contracting States:
**AT BE CH DE DK ES FR GB GR IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Lawson, Kevin Robert**
**High Stile, Piddington Lane**
**Piddington, High Wycombe, Bucks(GB)**
Inventor: **Biggin, Vincent Avery**
**53 Wessex Gardens**
**Twyford, Berkshire(GB)**

(74) Representative: **Ricks, Michael James et al**
**Imperial Chemical Industries PLC Legal Department: Patents PO Box 6 Bessemer Road**
**Welwyn Garden City Herts, AL7 1HD(GB)**

(54) **Pyrimidine derivatives.**

(57) A pyrimidine derivative of formula (I):-

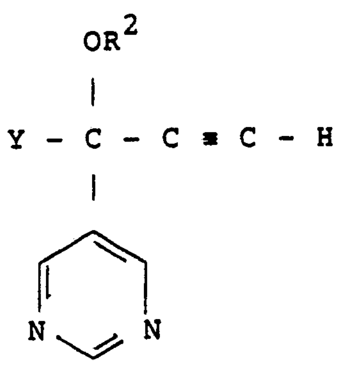
(I)

wherein Y an organic radical and $R^2$ is hydrogen, an alkyl group containing from 1 to 4 carbon atoms, an alkenyl group containing from 3 to 4 carbon atoms, or an acylate of an alkyl carboxylic acid containing from 2 to 4 carbon atoms or of an aryl carboxylic acid is useful as an intermediate in the manufacture of pyrimidine plant growth regulators via reaction with a phenyl halide. The compound of formula (I) wherein $R^2$ is hydrogen may be prepared by reacting a ketone of general formula (IV):-

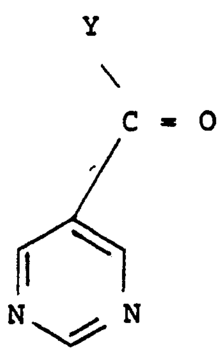
(IV)

with the reaction product of (a) $Z - C \equiv C - H$ wherein Z is hydrogen or a protective group, and (b) an organometallic compound; and (ii) subsequently removing the protective group, Z, where it is present.

EP 0 382 368 A1

## PYRIMIDINE DERIVATIVES

The present invention relates to pyrimidine derivatives, to the manufacture of pyrimidine derivatives and in particular to the manufacture of pyrimidine derivatives suitable as intermediates for the manufacture of pyrimidine-based plant growth regulators.

In co-pending European Patent Application No 88306768.8 there are disclosed pyrimidine derivatives useful as plant growth regulators and having the general formula (a):-

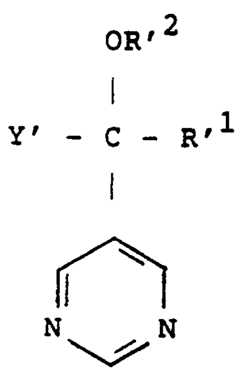

(a)

and stereoisomers thereof, wherein $Y'$ is optionally substituted cyclopropyl or optionally substituted 1-methylcyclopropyl or is the group:

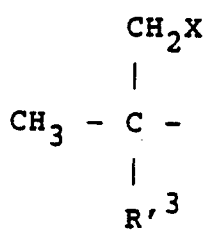

wherein $R'^3$ is hydrogen or methyl; $X'$ is hydrogen or halogen; $R'^1$ is inter alia a group:

$-C \equiv C-A'$

wherein $A'$ is an optionally substituted phenyl group; and $R'^2$ is hydrogen, an alkyl group containing from 1 to 4 carbon atoms, an alkenyl group containing from 3 to 4 carbon atoms or an alkynyl group containing from 3 to 4 carbon atoms and agrochemically acceptable salts, esters and metal complexes of the compounds of formula (a) wherein $R'2$ is hydrogen.

In co-pending British Patent Application No 8814026.4 there are disclosed pyrimidine derivatives useful as plant growth regulators and having the general formula (b):-

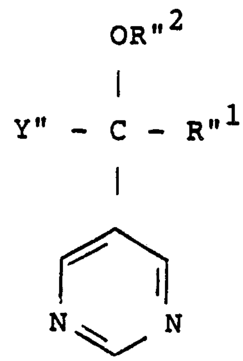

(b)

and stereoisomers thereof, wherein $Y''$ is an optionally substituted alkyl group of formula:

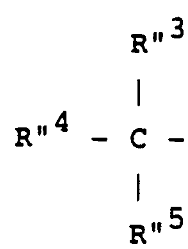

wherein $R''^3$, $R''^4$ and $R''^5$, which may be the same or different, are separately hydrogen or an optionally substituted alkyl group, provided that (a) no more than one of $R''^3$, $R''^4$ and $R''^5$ is hydrogen and that (b) $R''^3$, $R''^4$ and $R''^5$ are not all methyl or halomethyl at the same time and that (c) the group $Y''$ contains a total of from 4 to 10 alkyl carbon atoms; $R''^1$ is inter alia a group:

$-C \equiv C-A''$

wherein $A''$ is an optionally substituted phenyl group; and $R''^2$ is hydrogen, an alkyl group containing from 1 to 4 carbon atoms, an alkenyl group containing from 3 to 4 carbon atoms or an alkynyl group containing from 3 to 4 carbon atoms and agrochemically acceptable salts, esters and metal complexes of the compounds of formula (b) wherein $R''2$ is hydrogen.

The above applications disclose that the pyrimidine derivatives may be made by a process wherein an intermediate of general formula (c):

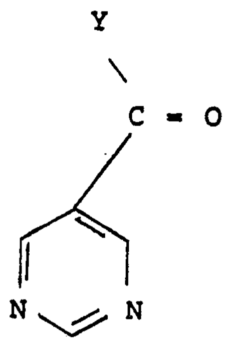

(c)

is reacted with an organometallic compound based on an organic group $R^1$ combined with a suitable metal, for example lithium, magnesium, titanium or zirconium and $R^1$ corresponds to $R'^1$ in the case of European Patent Application No 88306768.8 and $R''^1$ in the case of British Patent Application No 8814026.4.

Whilst the above process is generally suitable for the preparation of phenyl pyrimidine derivatives such as those of formula (a) and (b) above, yields may be poor when the phenyl alkene group is substituted in the phenyl ring by certain groups, and in particular by certain electron-withdrawing groups. Furthermore, when it is desired to prepare a range of compounds having different substitution on the phenyl ring, A, (where $R^1$ is - C ≡ C - A ), it is necessary to prepare a corresponding range of organometallic compounds $R^1M$.

The present invention seeks to provide a common intermediate for the simple, one-step preparation of compounds such as those of general formula (a) and (b) which differ in the substitution present in the phenyl ring (A).

According to the present invention there is provided a pyrimidine derivative of formula (I):-

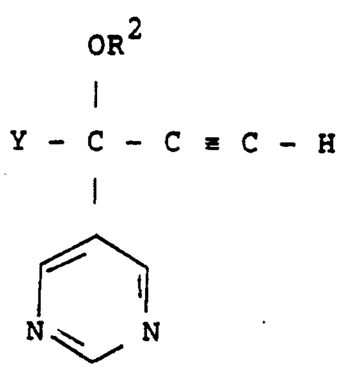

(I)

and stereoisomers thereof wherein Y an organic radical and $R^2$ is hydrogen, an alkyl group containing from 1 to 4 carbon atoms, an alkenyl group containing from 3 to 4 carbon atoms, or an acylate of an alkyl carboxylic acid containing from 2 to 4 carbon atoms or of an aryl carboxylic acid.

The compounds of the invention contain one or more chiral centres. Such compounds are generally obtained in the form of racemic mixtures. However, these and other mixtures can be separated into the individual isomers by methods known in the art, and this invention embraces such isomers.

Y is preferably an optionally substituted cyclopropyl or optionally substituted 1-methylcyclopropyl or is the group:

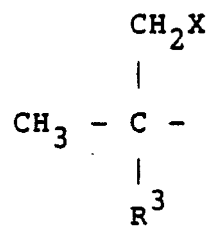

(II)

wherein $R^3$ is hydrogen or methyl and X is hydrogen or halogen.

Optional substituents which may be present in the group Y when it is is optionally substituted cyclopropyl or optionally substituted 1-methylcyclopropyl include lower alkyl (for example $C_1$ to $C_4$ alkyl) and halogen, especially monohalogen, for example chlorine or fluorine.

Where X in the compound of formula (II) is halogen, it is preferably chlorine or fluorine.

Alternatively, Y may be a group of general formula (III):

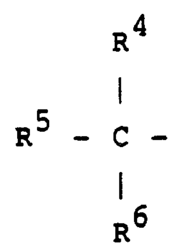

(III)

wherein $R^4$, $R^5$ and $R^6$, which may be the same or different, are separately hydrogen or an optionally substituted alkyl group, provided that (a) no more than one of $R^4$, $R^5$ and $R^6$ is hydrogen and that (b) $R^4$, $R^5$ and $R^6$ are not all either methyl or halomethyl at the same time and that (c) the group of general formula (III) contains a total of from 4 to 10 alkyl carbon atoms;

Optional substituents which may be present in the groups $R^4$, $R^5$ and $R^6$ include halogen, especially chlorine and fluorine.

A preferred group of formula (III) is t-pentyl.

$R^2$ is preferably hydrogen or methyl or is an acetate or benzoate group.

According to a further aspect of the present

invention there is provided a process for the manufacture of a compound of the general formula (I) above wherein Y has any of the meanings given above and $R^2$ is hydrogen, which comprises the steps of:

(i) reacting a ketone of general formula (IV):-

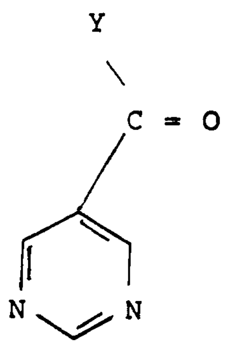

(IV)

with the reaction product of

(a) a compound of formula (V):-

Z - C ≡ C - H (V)

wherein Z is hydrogen or a protective group, and

(b) an organometallic compound;

and

(ii) subsequently removing the protective group, Z, where it is present.

Compounds of general formula (I) wherein $R^2$ is other than hydrogen can conveniently be prepared by reacting the corresponding compound of formula (I) above wherein $R^2$ is hydrogen with a suitable electrophile in the presence of a base. Examples of suitable electrophiles include the appropriate halide, acid chloride or acid anhydride.

The organometallic compound is suitably based on magnesium, lithium, titanium or zirconium, and may be for example ethyl magnesium bromide or n-butyl lithium. The titanium organometallic reagent is conveniently prepared by reaction of n-butyl lithium followed by chlorotitanium triisopropoxide. The zirconium organometallic compound is conveniently prepared by reaction of n-butyl lithium followed by chlorozirconium tributoxide.

The reaction of the compound of general formula (IV) in stage (i) above preferably takes place in the substantial absence of oxygen, for example in a nitrogen atmosphere.

The reaction preferably takes place in a suitable solvent, preferably an ether solvent, for example tetrahydrofuran, diethyl ether, dimethoxyethane, bis(2-methoxyethyl)ether or dimethoxymethane.

The reaction temperature is not critical, but is preferably in the range from -20° C to 40° C, for example from 0° C to ambient temperature. Superatmospheric or sub-atmospheric pressures may be used if desired, but the reaction preferably takes place at ambient pressure.

It is preferred that Z is a protective group. Suitable protective groups will occur to those skilled in the art as will the associated method of removing the protective group. Thus the protective group, Z, may for example be an organosilyl group. Suitable organosilyl protective groups include trialkyl silyl groups, for example the trimethylsilyl group. As a further example of a suitable protective group there may be mentioned a formaldehyde protective group such as that described by Bumagin, Ponomaryov and Beletskaya in USSR Synthesis (9), 728-9 (1984).

The compound of formula (V) may be prepared by methods known in the art, for example by the reaction of an acetylene derivative, for example an acetylene salt, with the corresponding halosilane or with a source of formaldehyde respectively.

The subsequent removal of the protective group, Z, may be accomplished by methods well known in the art for that protective group. For example a silyl protective group, Z, may be removed under the action of potassium carbonate in a suitable solvent such as methanol. Other suitable methods are described in "Silicon in Organic Synthesis" by Ernest W Colvin, Butterworths 1981, ISBN 0 408 10831 at page 165 and in the references cited therein. A formaldehyde protective group may be removed by oxidation as described in the USSR Synthesis publication noted above.

The Compound of formula IV may be prepared as described for example in United States Patent No 4,713,456.

Compounds of general formula (I) above are especially suitable as intermediates for the preparation of pyrimidine derivative plant growth regulators such as those disclosed in European Patent Application No 88306768.8 and British Patent Application No 8814026.4 wherein the single intermediate (I) can be used to prepare a range of products differing in the substitution of the phenyl ring.

Thus according to a further aspect of the present invention there is provided a process for the manufacture of a pyrimidine derivative of general formula (VI):-

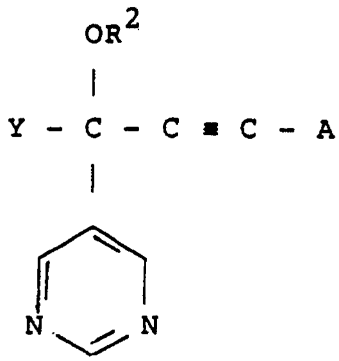
(VI)

and stereoisomers thereof wherein Y and $R^2$ have the meanings given previously, and A is an optionally substituted phenyl group which comprises reacting the intermediate of formula (I):-

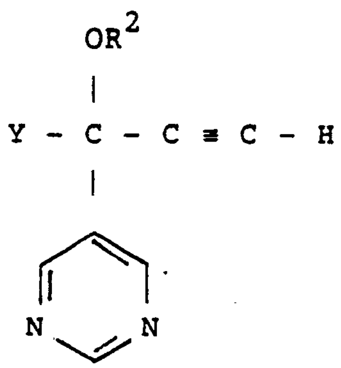
(I)

with an optionally substituted phenyl halide.

The further aspect of the present invention provides a simple one-step reaction from the common intermediate (I) to a pyrimidine derivative of formula (VI) using commonly available phenyl halides as reactants.

Preferably the phenyl halide is a phenyl iodide. Preferably the reaction takes place according to the known Heck reaction or a variant thereof (as described for example in Chemistry and Industry, 1987, (17), p 612; H M Colquhoun).

Suitable substituents which may be present in the phenyl group A include one or more substituents selected from halogen, for example chlorine, bromine or fluorine; alkyl (for example lower alkyl); cycloalkyl (for example cycloalkyl containing from 3 to 6 carbon atoms); alkoxy, for example lower alkoxy; haloalkyl, for example lower haloalkyl; lower alkoxy carbonyl (for example $-CO.CH_3$); nitro; amino, formamido, acetamido, dialkysulphamoyl and cyano. The term "lower" as applied to the above groups indicates that the group contains from 1 to 6 and preferably from 1 to 4 carbon atoms.

However, it is advantage of the present invention that the yield of desired product is not critically dependent on the nature of the substitution of the phenyl ring, and the scope of the invention should not therefore be seen as being limited to the above list of suitable substituents.

The invention is illustrated by the following Examples in which all parts and percentages are by weight.

## EXAMPLE 1

This example illustrates the preparation of 3-hydroxy-4-methyl-3-pyrimidin-5-ylpent-1-yne, used as a common intermediate of general formula (I) for the preparation of 1-[optionally substituted]phenyl-3-hydroxy-4-methyl-3-pyrimidin-5-ylpent-1-yne derivatives of general formula (VI).

### Stage 1

Preparation of 3-hydroxy-4-methyl-3-pyrimidin-5-yl-1-trimethylsilylpent-1-yne.

A solution of ethyl magnesium bromide (33ml of a solution which was 3M in tetrahydrofuran; 0.1mol) was added to a stirred, cooled solution of ethynyltrimethylsilane (9.8g,0.1mol) in dry tetrahydrofuran (150ml) under nitrogen. The rate of addition was adjusted so as to maintain the reaction temperature below 30°C. After stirring for 45 minutes, 2-methyl-1-pyrimidin-5-ylpropan-1-one (15g, 0.1mol) in dry tetrahydrofuran (30ml) was slowly added. The mixture was allowed to react for 2.8 hours before quenching with dilute aqueous HCl. Extraction with ethyl acetate, drying (over sodium sulphate) and concentration under reduced pressure gave the title compound as white crystals (20.3g). The product was further purified by recrystallisation from hexane to give a solid of melting point 116.6°C.

### Stage 2

Preparation of 3-hydroxy-4-methyl-3-pyrimidin-5-ylpent-1-yne.

Potassium carbonate (4g) was added to the product of stage 1 (19.5g, 0.11mol) in methanol (100ml) and the mixture was stirred for 3.3 hours. The mixture was then partitioned between ether and water. The ether layer was separated, dried over sodium sulphate, and concentrated under reduced pressure to give the title compound as an almost colourless solid (11.2g). The product was

further purified by recrystallisation from hexane to give a solid of melting point 112.4° C. The product was further characterised by its NMR spectrum as follows:-

NMR (270MHz, $CDCL_3$) $\delta_H$: 0.89 (3H,d,J7Hz), 1.09 (3H,d,J7Hz), 2.10 (1H,m), 2.77 (1H,s), 3.62 (1H,s) 8.92 (2H,s), 9.12 (1H,s)

## EXAMPLE 2

This example illustrates the general method for the coupling of [optionally substituted]-phenyliodides with the intermediate prepared in Example 1 to prepare a compound of general formula (VI).

Preparation of 3-hydroxy-4-methyl-3-pyrimidin-5-yl-1-(3-trifluoromethylphenyl)-pent-1-yne

3-hydroxy-4-methyl-3-pyrimidin-5-ylpent-1-yne (1g, 6mmol), prepared as in Example 1, 3-iodobenzenetrifluoride (1.6g, 6mmol), triethylamine (910µl, 6.6mmol), copper-I-iodide (60mg), and bis-(triphenylphosphine)palladium-II-chloride (60mg) were brought together in dry acetonitrile (20ml) and the mixture was heated at 65° C under nitrogen. After 1 hour, the mixture was cooled, quenched in dilute aqueous HCl, and extracted with ether (3 x 15ml). The combined organic extracts were washed with dilute aqueous ammonia, water, and brine before being dried over magnesium sulphate and concentrated under reduced pressure to give a red oil (1.95g). Flash chromatography on silica eluting with hexane-ethyl acetate (2:1) gave the title compound (1.28g) as off-white crystals of melting point 115.5-117° C.

## EXAMPLES 3 TO 11

The general method of Example 2 was used to prepare compounds of general formula (VI) by reaction of the intermediate of Example 1 with the appropriate optionally substituted phenyl iodide. The products were characterised by their melting point or by their NMR spectrum if they were not solids:-

3-hydroxy-4-methyl-3-pyrimidin-5-yl-1-(2-trifluoromethylphenyl)-pent-1-yne

$^1H$ NMR (270MHz,$CDCL_3$): δ0.98 (3H,d,J7Hz), 1.16 (3H,d,J7Hz), 2.13-2.28 (1H,m), 2.78 (1H,vbs), 7.46-7.74 (4H,m), 9.00 (2H,s), 9.19 (1H,s)

1-(4-aminophenyl)-3-hydroxy-4-methyl-3-pyrimidin-5-ylpent-1-yne, melting point 143-143.5° C.

3-hydroxy-4-methyl-3-pyrimidin-5-yl-1-(2-trifluoromethoxyphenyl)-pent-1-yne

NMR (270MHz,$CDCL_3$): δ0.94 (3H,d,J7Hz), 1.15 (3H,d,J7Hz) 2.12-2.27 (1H,m), 3.47 (1H,vbs), 7.25-7.55 (4H,m), 9.00 (2H,s), 9.16 (1H,s)

1-(2-aminophenyl)-3-hydroxy-4-methyl-3-pyrimidin-5-ylpent-1-yne.

$^1H$ NMR (270MHz,$CDCL_3$): δ0.98 (3H,d,J7Hz), 1.14 (3H,d,J7Hz), 2.12-2.28 (1H,m), 3.08 (1H,bs), 4.14 (2H,vbs), 6.69-6.74 (2H,m), 7.14-7.32 (2H,s), 9.00 (2H,s), 9.18 (1H,s)

1-(3-chlorophenyl)-3-hydroxy-4-methyl-3-pyrimidin-5-ylpent-1-yne, melting point 90.5-92.5° C.

1-(3-fluorophenyl)-3-hydroxy-4-methyl-3-pyrimidin-5-ylpent-1-yne.

$^1H$ NMR (270MHz,$CDCL_3$): δ0.95 (3H,d,J7Hz), 1.12 (3H,d,J7Hz), 2.18 (1H,m), 3.52 (1H,s), 7.05-7.38 (4H,m), 9.00 (2H,s), 9.17 (1H,s)

3-hydroxy-4-methyl-1-(4-nitrophenyl)-3-pyrimidin-5-ylpent-1-yne

$^1H$ NMR (270MHz,$CDCL_3$): δ0.97 (3H,d,J7Hz), 1.12 (3H,d,J7Hz), 2.21 (1H,s) 3.68 (1H,s) 7.63 (2H,d,J8Hz), 8.15 (2H,d,J8Hz), 9.10 (2H,s), 9.29 (1H,s)

3-hydroxy-1-(3-methoxyphenyl)-4-methyl-3-pyrimidin-5-ylpent-1-yne

$^1H$ NMR (270MHz,$CDCL_3$): δ0.96 (3H,d,J7Hz), 1.14 (3H,d,J7Hz), 2.19 (1H,s), 2.62 (1H,s), 3.82 (3H,s), 6.90-7.32 (4H,m), 9.3 (3H,s)

3-hydroxy-4-methyl-3-pyrimidin-5-yl-1-(4-trifluoromethylphenyl)-pent-1-yne.

$^1H$ NMR (270MHz,$CDCL_3$): δ0.97 (3H,d,J7Hz), 1.13 (3H,d,J7Hz), 2.11 (1H,m), 3.00 (1H,s), 7.55-7.66 (4H,m), 9.00 (2H,s), 9.20 (1H,s)

## EXAMPLE 12

1-(4-dimethylsulfamoylphenyl)-3-hydroxy-4-methyl-3-pyrimidin-5-ylpent-1-yne was prepared using the general method of Example 2. The 4-dimethylsulfamoyl-1-iodobenzene starting material was prepared as follows:-

To a stirred solution of 4-iodobenzenesulfonyl chloride (5g,16.5mmol) in dichloromethane (50ml) at 0° C under nitrogen was added a mixture of triethylamine (1.67g,16.5mmol), dimethylamine (approximately 1g,excess), and 4-dimethylaminopyridine (0.1g) in dichloromethane (10ml). After 2.5 hours, thin layer chromatography indicated the absence of starting sulfonyl chloride. The solution was washed twice with water, dried over sodium sulphate, and concentrated under reduced pressure to give the desired product as a pale yellow solid (5g), melting point 127-130.6° C.

The 1-(4-dimethylsulfamoylphenyl)-3-hydroxy-4-methyl-3-pyrimidin-5-ylpent-1-yne product was

characterised by its NMR spectrum:
$^1$H NMR (270MHz,$CDCL_3$): δ0.98 (3H,d,J7Hz), 1.13 (3H,d,J7Hz), 2.14-2.30 (1H,m), 2.73 (6H,s), 3.28 (1H,vbs), 7.64 (2H,d,J7Hz), 7.77 (2H,d,J7Hz), 9.21 (3H,vbs)

## EXAMPLE 13

This example illustrates the preparation of 4,4-dimethyl-3-hydroxy-3-pyrimidin-5-ylhex-1-yne, used as a common intermediate for the preparation of 1-[optionally substituted]phenyl-4,4-dimethyl-3-hydroxy-3-pyrimidin-5-ylhex-1-yne derivatives of general formula (VI).

### Stage 1

Preparation of 2,2-dimethyl-1-pyrimidin-5-ylbutan-1-one

To a stirred solution of lithium hexamethyldisilazide (22ml of a 1M solution in tetrahydrofuran; 22mmol) at -78 °C under nitrogen was slowly added 2-methyl-1-pyrimidin-5-ylpropan-1-one (3.04g, 20mmol) in dry tetrahydrofuran (5ml). After stirring for 30 minutes, iodoethane (1.76ml, 22mmol) was added in one portion. The mixture was allowed to warm to room temperature then heated under reflux for 2 hours. The reaction was quenched with saturated aqueous ammonium chloride and extracted with ethyl acetate (2 x 50ml). The combined organic extracts were washed with dilute aqueous HCl, saturated aqueous bicarbonate, and brine, before being dried over magnesium sulphate and concentrated under reduced pressure to give a red oil (2.5g). Flash chromatography eluting with hexane / ethyl acetate (2:1) gave the title compound (1.65g) as a yellow oil contaminated with 30% 2-methyl-1-ethoxy-1-pyrimidin-5-ylprop-1-ene. This mixture was used without further purification in Stage 2.

### Stage 2

Preparation of 4,4-dimethyl-3-hydroxy-3-pyrimidin-5-ylhex-1-yne

To a stirred solution of ethynyltrimethylsilane (1.24ml, 8.8mmol) in dry tetrahydrofuran (10ml) at 0 °C under nitrogen was added ethyl magnesium bromide (2.9ml of a solution 3M in tetrahydrofuran; 8.8mmol). When effervescence had ceased (40 minutes) crude 2,2-dimethyl-1-pyrimidin-5-ylbutan-1-one (prepared in stage 1) (1.5g, 8mmol) in dry tetrahydrofuran (5ml) was added. After 90 minutes, gas/liquid chromatography indicated complete consumption of starting material and the reaction was quenched with saturated aqueous ammonium chloride. The product was extracted with ethyl acetate (3 x 20ml) and the organic layers were combined, washed with dilute aqueous HCl, saturated aqueous sodium bicarbonate, and brine before being dried over magnesium sulphate and concentrated under reduced pressure to give a yellow oil (1.98g). A portion of this product (1.8g) was dissolved in methanol (10ml) and treated with anhydrous potassium carbonate (400mg). After stirring for 48 hours, the mixture was diluted with ethyl acetate, washed twice with water and brine before being dried over magnesium sulphate and concentrated under reduced pressure to give an oil (1.3g). Flash chromatography eluting with hexane / ethyl acetate (2:1) gave the title compound (950mg) as white crystals, melting point 99-101 °C. The impurity, 2-methyl-1-ethoxy-1-pyrimidin-5-ylprop-1-ene (370mg) was retrieved as an oil.

## EXAMPLE 14

This example illustrates the preparation of 4,4-dimethyl-3-hydroxy-3-pyrimidin-5-ylpent-1-yne used in the preparation of 4,4-dimethyl-3-hydroxy-1-[optionally substituted]-phenyl-3-pyrimidin-5-yl-pent-1-yne compounds used as a common intermediate of general formula (I).

To a stirred solution of ethynyltrimethylsilane (6.0g,61mmol) in dry tetrahydrofuran (100ml) under argon, cooled by a water bath, was slowly added a solution of ethyl magnesium bromide (3M in tetrahydrofuran, 20.5ml,61.5mmol). After 1.75 hours, 2,2-dimethyl-1-pyrimidin-5-ylpropan-1-one (10g,61mmol) was added, and the resulting solution was stirred for a further 3.5 hours. The reaction mixture was partitioned between ethyl acetate and dilute aqueous HCl, and the organic layer was separated, washed with water, dried over sodium sulphate and concentrated under reduced pressure to give a buff solid (14.1g). A portion of this solid (13.2g) was dissolved in methanol (120ml) and the resulting solution was stirred and treated with anhydrous potassium carbonate (3g). After 3.5 hours, the solvent was removed under reduced pressure and the residue partitioned between dichloromethane and water. The organic layer was separated, and filtered through phase separation paper. Concentration under reduced pressure gave 4,4-dimethyl-3-hydroxy-3-pyrimidin-5-ylpent-1-yne as a buff solid (7.13g), melting point 163.9 °C.

EXAMPLE 15

This example illustrates the general method for the coupling of the alkyne intermediate prepared in Example 14 with an [optionally substituted] phenyl iodide.

Preparation of 1-(4-Bromophenyl)-4,4-dimethyl-3-hydroxy-3-pyrimidin-5-ylpent-1-yne

A stirred mixture of 4,4-dimethyl-3-hydroxy-3-pyrimidin-5-ylpent-1-yne (2.5g,13mmol), 4-iodobromobenzene (3.7g,13mmol), bis-(triphenylphosphine)palladium-II-chloride (0.1g), copper-I-iodide (0.1g), and triethylamine (2.5ml,18mmol) in dry acetonitrile (30ml) was heated at 50 °C under a nitrogen atmosphere for 2.5 hours. The mixture was cooled and partitioned between dilute aqueous ammonia and ethyl acetate. The organic layer was separated, washed with dilute aqueous ammonia and water, dried over sodium sulphate, and concentrated under reduced pressure to give a white solid, which was triturated with hexane and dried to give the title product (3.13g), melting point 161.3 °C

## Claims

1. A pyrimidine derivative of formula (I):-

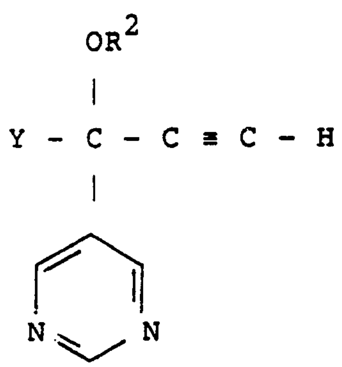

(I)

and stereoisomers thereof wherein Y an organic radical and $R^2$ is hydrogen, an alkyl group containing from 1 to 4 carbon atoms, an alkenyl group containing from 3 to 4 carbon atoms, or an acylate of an alkyl carboxylic acid containing from 2 to 4 carbon atoms or of an aryl carboxylic acid.

2. A pyrimidine derivative according to claim 1 wherein Y is

(i) an optionally substituted cyclopropyl group;

(ii) an optionally substituted 1-methylcyclopropyl group;

(iii) the group:

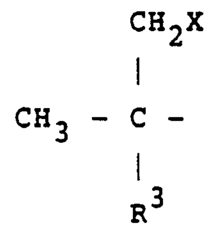

(II)

wherein $R^3$ is hydrogen or methyl and X is hydrogen or halogen; or

(iv) a group of formula (III):

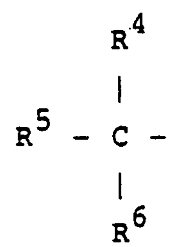

(III)

wherein $R^4$, $R^5$ and $R^6$, which may be the same or different, are separately hydrogen or an optionally substituted alkyl group, provided that (a) no more than one of $R^4$, $R^5$ and $R^6$ is hydrogen and that (b) $R^4$, $R^5$ and $R^6$ are not all either methyl or halomethyl at the same time and that (c) the group of general formula (III) contains a total of from 4 to 10 alkyl carbon atoms;

3. A pyrimidine derivative according to claim 1 wherein Y is t-pentyl.

4. A pyrimidine derivative according to any of claims 1 to 3 wherein $R^2$ is hydrogen or methyl or is an acetate or benzoate group.

5. A process for the manufacture of a compound of the formula (I) as defined in claim 1 wherein $R^2$ is hydrogen, which comprises the steps of:

(i) reacting a ketone of general formula (IV):-

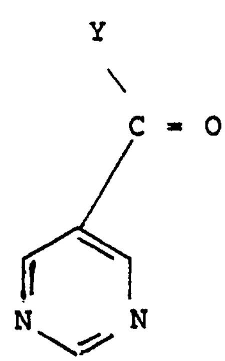

(IV)

with the reaction product of

(a) a compound of formula (V):-

$Z-C\equiv C-H$ (V)

wherein Z is hydrogen or a protective group, and

(b) an organometallic compound;

and

(ii) subsequently removing the protective group, Z, where it is present.

6. A process according to claim 5 wherein the organometallic compound is based on magnesium, lithium, titanium or zirconium.

7. A process according to claim 5 or 6 wherein the protective group, Z, is a trialkyl silyl group.

8. A process for the manufacture of a pyrimidine derivative of general formula (VI):-

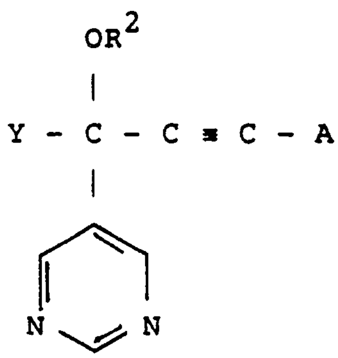

(VI)

and stereoisomers thereof wherein Y and $R^2$ are as defined in claim 1 and A is an optionally substituted phenyl group which comprises reacting an intermediate of formula (I) as defined in claim 1 with an optionally substituted phenyl halide.

9. A process according to claim 8 wherein the optionally substituted phenyl halide is an optionally substituted phenyl iodide and the reaction takes place according to the Heck reaction.

European Patent Office

# EUROPEAN SEARCH REPORT

Application number: EP 90300650.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | EP - A2 - 0 272 813 (ICI) * Claims 1,4,10 * -- | 1,5,8 | C 07 D 239/26 // A 01 N 43/54 |
| A | EP - A2 - 0 295 839 (ICI) * Claims 1,15 * -- | 1,5 | |
| D,P, A | EP - A1 - 0 304 171 (ICI) * Claims 1,7 * ---- | 1,5 | |
| | | | TECHNICAL FIELDS SEARCHED (Int Cl.5) |
| | | | C 07 D 239/00 |

The present search report has been drawn up for all claims

| Place of search | Date of completion of the search | Examiner |
|---|---|---|
| VIENNA | 04-04-1990 | LUX |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document